(19) Europäisches Patentamt
European Patent Office
Office européen des brevets

(11) Publication number: 0 254 516 B1

(12) **EUROPEAN PATENT SPECIFICATION**

(45) Date of publication of patent specification: **05.01.94**

(51) Int. Cl.[5]: **C07K 7/40**, A61K 37/02

(21) Application number: **87306405.9**

(22) Date of filing: **20.07.87**

The file contains technical information submitted after the application was filed and not included in this specification

# (54) Novel Peptides.

(30) Priority: **21.07.86 DK 3470/86**

(43) Date of publication of application:
**27.01.88 Bulletin 88/04**

(45) Publication of the grant of the patent:
**05.01.94 Bulletin 94/01**

(84) Designated Contracting States:
**AT BE CH DE ES FR GB GR IT LI LU NL SE**

(56) References cited:
**EP-A- 0 046 979**
**EP-A- 0 140 084**
**EP-A- 0 194 864**

(73) Proprietor: **NOVO NORDISK A/S**
**Novo Allé**
**DK-2880 Bagsvaerd(DK)**

(72) Inventor: **Markussen, Jan**
**7, Kikudbakken**
**DK-2730 Herlev(DK)**
Inventor: **Norris, Kjeld**
**34, Ahlmanns Allé**
**DK-2900 Hellerup(DK)**
Inventor: **Langkjaer, Liselotte**
**22A, Kroyersvej**
**DK-2930 Klampenborg(DK)**

(74) Representative: **Noergaard, Torsten et al**
**c/o Novo Nordisk A/S**
**Novo Allé**
**DK-2880 Bagsvaerd (DK)**

EP 0 254 516 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

### BACKGROUND OF THIS INVENTION

The present invention relates to novel insulin compounds and to novel injectable solutions having prolonged insulin action.

In the treatment of diabetes mellitus, many varieties of insulin preparations have been suggested and used. Some of the preparations are fast acting and other preparations have more or less prolonged actions. Such a prolonged action may be obtained by administering the insulin as a suspension of insulin crystals. The crystalline preparations can be obtained by crystallization of insulin in the presence of zinc (such as Lente™, see Schlichtkrull: Insulin Crystals, Chemical and Biological Studies on Insulin Crystals and Insulin Zinc Suspensions, Munksgaard, 1958) or by crystallization of insulin in the presence of zinc and protamine (such as NPH-insulin, see Rep.Steno Mem.Hosp. 1 (1946), 60).

One disadvantage in the use of the known suspensions of zinc insulin crystals or of zinc protamine insulin is the necessity of shaking the vial in order to ensure that the correct amount of insulin is being injected and to ensure that the concentration of insulin in the vial remains constant throughout its use. In Penfill™ cartridges where air must be absent, prolonged acting insulin suspensions require the incorporation of a solid body in the cartridge to enable agitation. The shaking of insulin suspensions and insulin solutions with air is in itself an undesirable process, as insulin has a tendency to denature under formation of fibrills at water-air interfaces. Consequently, solutions of insulins with prolonged action are desirable.

Solutions of insulin derivatives having a prolonged action were obtained from insulin that had been modified in its amino groups by reaction with phenylisocyanate (so-called Iso-insulin, see Hallas-Moeller: Chemical and Biological Insulin Studies based upon the Reaction between Insulin and Phenylisocyanate, Copenhagen 1945). Similarly, Al,B29-di-Boc substituted insulin (Boc designates tertiary butyloxycarbonyl) was reported to show a prolonged insulin action after subcutaneous administration (see Geiger & Enzmann in: Proinsulin, Insulin, C-peptide; Proceedings of the Symposium on Proinsulin, Insulin and C-Peptide, Tokushima 1978; Amsterdam-Oxford 1979, 306 - 310). The Al,B29-di-Boc substituted insulin was found to exhibit a too slightly prolonged action to be clinically useful.

Solutions of unmodified insulins require large amounts of zinc ions (for example, 0.4 - 1 mg/U insulin) in order to exhibit a prolonged action (see J.Pharmacol. 55 (1935), 206). Injection of such large doses of zinc ions will probably cause pain and such solutions have, therefore, never been used in therapy.

The isoelectric point of insulin is about 5.5 and attempts have been made to decrease the solubility of insulin derivatives at neutral pH by shifting the isoelectric point upwards, for example, through additions, in the N-terminus of the B-chain, of basic amino acids like lysine or arginine (see, for example, German Offenlegungsschrift No. 2,042,299) or with the basic dipeptide arginyl-arginine (see Geiger & Enzmann cited above). However, near its isoelectric point the solubility of $Arg^{B(-1)}$-$Arg^{B0}$ insulin was much higher than that of the parent insulin.

Japanese patent application No. 55-144032 relates to analogues to human insulin wherein the B30-amino acid has been replaced by an amino acid having at least five carbon atoms, and amides and esters thereof. These insulin analogues were to be used in patients who had developed antibodies against mammalian insulins. In the Japanese patent application, six specific compounds are described, none of which were stated to have prolonged action. No specific injectable preparations are described in the Japanese patent application.

European patent application No. 84108442.9 relates to insulin analogues wherein a basic, organic group is attached to the B30-amino acid thereby introducing a positive charge at neutral pH. In these analogues, the B30-amino acid is neutral and, preferably, threonine as in human insulin. German patent application No. 3,327,709.5 relates to a suspension of crystals of the derivatives described in the above-noted European patent application as well as an aromatic hydroxy compound. German patent application No. 3,326,473.2 relates to a medicament containing a mixture of insulin compounds, of which at least one is described in the above-noted European patent application.

European patent application publication No. 194.864 claiming priority from 12 March 1985 relates to insulin derivatives in which the C-terminal B30 residue always is blocked by an amido or ester group, and in which the A21 amino acid always is asparagine like in human insulin.

### BRIEF STATEMENT OF THE INVENTION

The present invention comprises novel analogs of human insulin that differ from human insulin by:

a) optional presence of an amide or ester residue on the C-terminal carboxyl group of the B-chain,

b) having at least one charge more than human insulin at pH 7, preferably not more than 4 charges more than human insulin at pH 7,

c) that the C-terminal asparagine residue of the A-chain, $Asn^{A21}$, is substituted with any other naturally occurring amino acid, which can be coded for by nucleotide sequences,

d) if a change in charge is achieved by blocking of the carboxylic group in the B30 amino acid, the A21 amino acid residue is different from an asparagine residue.

The change in charge is achieved by substituting one or more of the amino acid residues compared with human insulin and, if desired, by the blocking of the carboxylic group in the B30 amino acid.

In specific, the compounds of interest to practice of this invention are characterizable as follows: One or more of the four glutamic acid residues at A4, A17, B13, B21 are replaced by another naturally occurring neutral amino acid residue, preferably a glutamine residue; and/or the threonine residue at B27 is instead a naturally occurring basic amino acid residue, preferably an L-arginine or L-lysine residue; and/or the threonine residue at B30 is replaced by one or two basic amino acid residues, one being preferred; the C terminal carboxylic group in the B chain may be protected, and the asparagine residue at A21 is substituted by an L amino acid residue.

The invention also comprises solutions of the below compounds of formula I, optionally containing a controlled level of zinc ions therein. The degree of prolongation of insulin action is enhanced and controlled thereby.

## DETAILED PRACTICE OF THIS INVENTION

It has surprisingly been found that injectable solutions with a combined short and prolonged insulin action can be made using, as the active ingredient, a single insulin derivative having the general formula I

```
A(1-3)-E1-A(5-6)-Cys-A(8-16)-E2-A(18-19)-Cys-W          (A-chain)
                  |                         |
                  S                         S
                  |                         |
                  S                         S                (I)
                  |                         |
      B(1-6)-Cys-B(8-12)-E3-B(14-18)-Cys
                                            |                (B-chain)
        R-Zn-Ym-Lys-Pro-X-B(26-22)-E4-Gly
```

wherein the letters A and B followed by figures in parentheses designate the peptide fragments of the A- and B-chains, respectively, indicated by the figures in parentheses, $E^1$, $E^2$, $E^3$ and $E^4$ are the same or different each representing a glutamic acid residue or a neutral amino acid residue which can be coded for by nucleotide sequences, X represents an L-threonine, L-arginine or L-lysine residue, Y and Z are the same or different and each represent an amino acid residue wherein any side chain amino group may be acylated and wherein any side chain hydroxy group may be alkylated, m and n are the same or different and each represent zero or one, R represents hydroxy or an amido or ester residue which blocks the C-terminal carboxyl group of the B-chain, and W represents an amino acid residue except asparagine, with the further proviso that at least one of the six amino acid residues $E^1$, $E^2$, $E^3$, $E^4$, W and X is different from the amino acid residues present at the corresponding positions in human insulin, and that at least one of the eight amino acid residues $E^1$, $E^2$, $E^3$, $E^4$, W, X, Y and Z and the group R are selected so that the compound of formula I has at least one charge more than human insulin at a pH value of 7.

A subgroup of compounds of formula I is novel compounds having the general formula I wherein the letters A and B followed by figures in parentheses designate the peptide fragments of the A- and B-chains, respectively, indicated by the figures in parentheses, $E^1$, $E^2$, $E^3$ and $E^4$ are the same or different each representing a glutamic acid residue or a neutral amino acid residue which can be coded for by nucleotide sequences, X represents an L-threonine, L-arginine or L-lysine residue, Y and Z are the same or different and each represent an amino acid residue wherein any side chain amino group may be acylated and

wherein any side chain hydroxy group may be alkylated, m and n are the same or different and each represent zero or one, R represents an amido or ester residue which blocks the C-terminal carboxyl group of the B-chain, and W is different from an asparagine residue.

Compared with human insulin, the change in charge is obtained by substituting the threonine residue in the B27-position with an arginine or lysine residue and/or by substituting any of the four glutamic acid residues in the A4-, A17-, B13-, and B21-position with a neutral amino acid residue, preferably with a glutamine residue. In addition, the C-terminal carboxyl group of the B-chain may be blocked by an ester group or amide group, thereby eliminating the negative charge of the carboxyl group. Furthermore, a positive charge may be introduced by a basic amino acid residue in the B30- and/or B31-position.

Since compounds of formula I can be applied in the clinic as solutions having a prolonged action, a decline in immunogenicity as compared to the commonly used suspensions of porcine or human insulins may occur.

The degree of prolongation can be enhanced and controlled by the addition of zinc ions.

Major parameters that control the degree of prolongation of the insulin effect are the concentration of zinc and the choice of the compound of formula I. The range for preferred zinc content extends from 0 to about 2 mg/ml, preferably from 0 to 200 $\mu$g/ml zinc with substitution in the B13 and/or B27 position and preferably from about 20 to 200 $\mu$g/ml with other analogs in a preparation containing 240 nmole of a compound of formula I per ml. Using other concentrations of the compound of formula I, the content of zinc is to be adjusted correspondingly.

The prolonged action of solutions of compounds of formula I in the presence of zinc ions is ascribed to the low solubility of such compounds at neutral pH.

The pH of the injectable solution of this invention should preferably be below the physiological pH, the upper limit being the pH where precipitation occurs. At the physiological pH value, compounds of formula I of this invention have a low solubility. Stable solutions containing about 240 nmole/ml of compounds of formula I per ml have been obtained at pH about 5.5. The upper limit depends upon the constituents of the solution, i.e. isotonikum, preservative and zinc concentration, and upon the choice of compound of formula I. There is no lower pH limit of the solutions and the chemical stability of the compounds of formula I where W is different from asparagine, is high, even at pH 3. The preferred pH range for the injectable solutions of this invention is from about 2.5 to 8.5, more preferred from about 4.5 to 8. Especially preferred are pH ranges about 2.5 to 5.5, most preferred about 3 to 4.5.

A further aspect of this invention is that it provides improved flexibility for the patients. With two aqueous solutions, one containing a compound of formula I and the other containing a zinc salt, the patient can obtain a desired degree of prolonged action and a desired profile by mixing the two solutions appropriately. Thus, the patient has, using two stock solutions, the possibility of choosing one action and profile for the morning injection and another action and profile for the evening injection. Preferably, the zinc solution of this invention contains between about 2 $\mu$g and 20 mg zinc per ml. Alternatively, both of the stock solutions may contain zinc, either in the same or different concentrations, and/or both the stock solutions may contain a compound of formula I, either the same or different compounds.

Preferably, the injectable solutions of this invention have a strength of between about 60 and 6000 nmole of the compound of formula I per ml.

When W is not L-asparagine it may be a neutral L-amino acid, for example valine, glutamine, isoleucine, leucine, phenylalanine, tyrosine, methionine or preferably glycine, serine, threonine, alanine or homoserine. W may be an acidic amino acid, viz. glutamic acid or preferably aspartic acid, or a basic amino acid, viz. lysine, arginine or preferably histidine.

The neutral amino acid ($E^1$ through $E^4$) is, for example, glycine, valine, isoleucine, leucine, phenylalanine, tyrosine, methionine or preferably asparagine, glutamine, alanine, serine or threonine.

Examples of R are ester moieties, for example, lower alkoxy, preferably methoxy, ethoxy and most preferred tertiary butoxy.

Furthermore, R can be a group of the general formula $-NR^1R^2$ wherein $R^1$ and $R^2$ are the same or different and each represents hydrogen or lower alkyl. Hereinafter the term "lower" designates that the group in question contains less than 7 carbon atoms, preferably less than 5 carbon atoms. In a preferred embodiment of this invention, R is $-NH_2$. Furthermore, R may be a lactam residue which preferably contains less than 8 atoms in the lactam ring, for example a lactam of a diaminocarboxylic acid.

In a preferred embodiment of this invention, R is uncharged.

According to one preferred embodiment of this invention, the amino acid residues designated Y and Z are residues from L-amino acids which are coded for by nucleotide sequences.

Any side chain amino group in the amino acid residues designated Y and Z may be acylated by an acid containing from 2 to 18 carbon atoms, preferably a fatty acid containing from 6 to 18 carbon atoms, for

example, lauric acid. Thus, $-Y_m-Z_n-R$ may be $-Lys(Lau)-NH_2$.

Examples of preferred alkylated hydroxy groups are methoxy, ethoxy and tertiary butoxy.

In one group of preferred compounds of formula I Y and/or Z is a basic amino acid residue wherein the side chain amino group optionally is acylated (m = 1).

In another group of preferred compounds of formula I n is zero and Y is a basic amino acid residue (m = 1).

In a further group of preferred compounds of formula I Y and Z are both basic amino acid residues (m = 1, n = 1).

Another preferred embodiment of this invention is preparations containing a compound of formula I wherein $E^1$, $E^2$, $E^3$ and/or $E^4$ is a glutamine residue, and/or X is Lys or Arg, and W is Gly, Ser, Thr, Ala, His, Asp or hSer, and within this subclass of compounds of formula I, a further preferred embodiment is preparations containing a compound of formula I wherein the group $-Y_m-Z_n-R$ is $-Thr-NH_2$ or $-Lys-NH_2$.

Specific preferred compounds of formula I are each of the following:

$Gly^{A21},Lys^{B27},Thr^{B30}-NH_2$ human insulin,
$Ser^{A21},Lys^{B27},Thr^{B30}-NH_2$ human insulin,
$Thr^{A21},Lys^{B27},Thr^{B30}-NH_2$ human insulin,
$Ala^{A21},Lys^{B27},Thr^{B30}-NH_2$ human insulin,
$His^{A21},Lys^{B27},Thr^{B30}-NH_2$ human insulin,
$Asp^{A21},Lys^{B27},Thr^{B30}-NH_2$ human insulin,
$Gly^{A21},Arg^{B27},Thr^{B30}-NH_2$ human insulin,
$Ser^{A21},Arg^{B27},Thr^{B30}-NH_2$ human insulin,
$Thr^{A21},Arg^{B27},Thr^{B30}-NH_2$ human insulin,
$Ala^{A21},Arg^{B27},Thr^{B30}-NH_2$ human insulin,
$His^{A21},Arg^{B27},Thr^{B30}-NH_2$ human insulin,
$Asp^{A21},Arg^{B27},Thr^{B30}-NH_2$ human insulin,
$Gln^{B13},Gly^{A21},Arg^{B27},Thr^{B30}-NH_2$ human insulin,
$Gln^{B13},Ser^{A21},Arg^{B27},Thr^{B30}-NH_2$ human insulin,
$Gln^{B13},Thr^{A21},Arg^{B27},Thr^{B30}-NH_2$ human insulin,
$Gln^{B13},Ala^{A21},Arg^{B27},Thr^{B30}-NH_2$ human insulin,
$Gln^{B13},His^{A21},Arg^{B27},Thr^{B30}-NH_2$ human insulin,
$Gln^{B13},Asp^{A21},Arg^{B27},Thr^{B30}-NH_2$ human insulin,
$Gln^{B13},Gly^{A21},Lys^{B27},Thr^{B30}-NH_2$ human insulin,
$Gln^{B13},Ser^{A21},Lys^{B27},Thr^{B30}-NH_2$ human insulin,
$Gln^{B13},Thr^{A21},Lys^{B27},Thr^{B30}-NH_2$ human insulin,
$Gln^{B13},Ala^{A21},Lys^{B27},Thr^{B30}-NH_2$ human insulin,
$Gln^{B13},His^{A21},Lys^{B27},Thr^{B30}-NH_2$ human insulin,
$Gln^{B13},Asp^{A21},Lys^{B27},Thr^{B30}-NH_2$ human insulin.

Another preferred embodiment of this invention is preparations containing a compound of formula I in which m is one, n is zero, Y is Thr, Ala or Ser, R is hydroxy, and W is Gly, Ser, Thr, Ala, His or Asp, and examples of such compounds are as follows:

$Ser^{A21},Lys^{B27}$ human insulin,
$Thr^{A21},Lys^{B27}$ human insulin,
$Ala^{A21},Lys^{B27}$ human insulin,
$His^{A21},Lys^{B27}$ human insulin,
$Asp^{A21},Lys^{B27}$ human insulin,
$Gly^{A21},Lys^{B27}$ human insulin,
$Ser^{A21},Arg^{B27}$ human insulin,
$Thr^{A21},Arg^{B27}$ human insulin,
$Ala^{A21},Arg^{B27}$ human insulin,
$His^{A21},Arg^{B27}$ human insulin,
$Asp^{A21},Arg^{B27}$ human insulin,
$Gly^{A21},Arg^{B27}$ human insulin,
$Gln^{A17},Ser^{A21},Arg^{B27}$ human insulin,
$Gln^{A17},Thr^{A21},Arg^{B27}$ human insulin,
$Gln^{A17},Ala^{A21},Arg^{B27}$ human insulin,
$Gln^{A17},His^{A21},Arg^{B27}$ human insulin,
$Gln^{A17},Asp^{A21},Arg^{B27}$ human insulin,
$Gln^{A17},Gly^{A21},Arg^{B27}$ human insulin,

$Gln^{A17}$,$Ser^{A21}$,$Gln^{B13}$ human insulin,
$Gln^{A17}$,$Thr^{A21}$,$Gln^{B13}$ human insulin,
$Gln^{A17}$,$Ala^{A21}$,$Gln^{B13}$ human insulin,
$Gln^{A17}$,$His^{A21}$,$Gln^{B13}$ human insulin,
$Gln^{A17}$,$Asp^{A21}$,$Gln^{B13}$ human insulin,
$Gln^{A17}$,$Gly^{A21}$,$Gln^{B13}$ human insulin,
$Arg^{B27}$,$Ser^{A21}$,$Gln^{B13}$ human insulin,
$Arg^{B27}$,$Thr^{A21}$,$Gln^{B13}$ human insulin,
$Arg^{B27}$,$Ala^{A21}$,$Gln^{B13}$ human insulin,
$Arg^{B27}$,$His^{A21}$,$Gln^{B13}$ human insulin,
$Arg^{B27}$,$Asp^{A21}$,$Gln^{B13}$ human insulin,
$Arg^{B27}$,$Gly^{A21}$,$Gln^{B13}$ human insulin,
$Gln^{A17}$,$Ser^{A21}$,$Lys^{B27}$ human insulin,
$Gln^{A17}$,$Thr^{A21}$,$Lys^{B27}$ human insulin,
$Gln^{A17}$,$Ala^{A21}$,$Lys^{B27}$ human insulin,
$Gln^{A17}$,$His^{A21}$,$Lys^{B27}$ human insulin,
$Gln^{A17}$,$Asp^{A21}$,$Lys^{B27}$ human insulin,
$Gly^{A17}$,$Gly^{A21}$,$Lys^{B27}$ human insulin,
$Gln^{B13}$,$Ser^{A21}$,$Lys^{B27}$ human insulin,
$Gln^{B13}$,$Thr^{A21}$,$Lys^{B27}$ human insulin,
$Gln^{B13}$,$Ala^{A21}$,$Lys^{B27}$ human insulin,
$Gln^{B13}$,$His^{A21}$,$Lys^{B27}$ human insulin,
$Gln^{B13}$,$Asp^{A21}$,$Lys^{B27}$ human insulin,
$Gly^{B13}$,$Gly^{A21}$,$Lys^{B27}$ human insulin.

In one group of preferred compounds of formula I, $E^2$ and $E^3$ is a glutamine residue.

In another group of preferred compounds of formula I, X is an arginine or lysine residue.

In a further group of preferred compounds of formula I, W is Gly, Ser, Thr, Ala, His, Asp or hSer.

As is well known in the art, not all of the amino acid residues in human insulin are essential for the insulin action.

Indeed, porcine insulin and bovine insulin which differs from human insulin in amino acid residues have been employed to treat diabetics. Considerable species to species variations exist in the insulin molecule. Thus, many amino acid residues in the human insulin molecule may be changed without undue diminution in insulin activity, including some residues influencing the isoelectric point of the molecule.

It is obvious that the groups designated $E^1$, $E^2$, $E^3$, $E^4$, R, W, X, Y and Z are to be selected so that the resulting compound of formula I is pharmaceutically acceptable.

In the known biphasic insulin preparations, it is common to combine fast acting, soluble insulin with prolonged acting, crystalline insulin in the same injection. Using compounds of formula I of this invention, a similar combined short and prolonged action can be obtained with a solution of a single compound of formula I. The ratio between short and long acting effect decreases as the concentration of zinc ions in the solution is increased.

Compounds of formula I may be prepared by a transpeptidation reaction in which a biosynthetic precursor compound having the correct insulin disulfide bridges and having the general formula II:

$$\begin{array}{l} X\text{-}B(26\text{-}22)\text{-}E^4\text{-}B(20\text{-}14)\text{-}E^3\text{-}B(12\text{-}1) \\ | \\ B(28\text{-}29)\text{-}(Q_q\text{-}T)_r\text{-}A(1\text{-}3)\text{-}E^1\text{-}A(5\text{-}16)\text{-}E^2\text{-}A(18\text{-}20)\text{-}W \end{array} \qquad (II)$$

wherein A and B followed by figures in parentheses designate the appropriate peptide fragments of the A- and B-chains, respectively, as indicated by the figures in parentheses, Q is a peptide chain with q amino acids, q is an integer from 0 to 33, T is Lys or Arg, r is zero or one, and $E^1$, $E^2$, $E^3$, $E^4$, W and X each are as defined above, is reacted with an amino compound of the general formula III:

$H\text{-}Y_m\text{-}Z_n\text{-}R$ (III)

wherein Y, Z, R, m and n each are as defined above, and wherein side chain amino groups and hydroxy

groups in Y and Z optionally are blocked with amino and hydroxy protecting groups, using trypsin or a trypsin like enzyme as a catalyst in a mixture of water and organic solvents analogously as described in U.S. Patent No. 4,343,898. When W is hSer, nucleotides coding for Met are introduced at the A21 site in the gene. In the protein expressed conversion of Met into hSer is accomplished by cyanogenebromide. Preferred compounds of formula III for use in this process are Thr-$NH_2$, Lys(Boc)-$NH_2$, Thr($Bu^t$)-$OBu^t$, Thr-$OBu^t$, Ala-$NH_2$ and Arg(Boc)-$NH_2$. Amino groups may be derivatized by acylation with a fatty acid. Hydroxy groups may be protected by alkylation. If Y and Z contain groups which are reversibly blocked by amino protecting groups, these groups may be removed at a later stage, after the amino protected intermediate has been separated from the trypsin or trypsin like enzyme. Of the trypsin like enzymes, lysyl endopeptidase from Achromobacter lyticus is useful.

The compound of formula II may be expressed in a host organism such as yeast similar to the description in European patent application publication No. 163,529 using a gene having the correct codons for the amino acids in question. The gene encoding the novel insulin derivative is then inserted into a suitable expression vector which when transferred to yeast is capable of expressing the desired compound. The product expressed is then isolated from the cells or the culture broth depending on whether it is secreted from the cells or not.

An example of a reversible amino protecting group is tertiary butoxycarbonyl and a reversible hydroxy protecting group is tertiary butyl. Such groups are removed under conditions which do not cause undesired alteration in the compound of formula I, for example, by trifluoroacetic acid.

Changes in the A4, A17, A21, B13, B21 or B27 position may conveniently be introduced by genetic engineering, leaving for trypsin catalyzed semisynthesis to introduce the desired C-terminal residue of the B-chain.

The advantage in introducing the additional positive charges within the frame of the 51 amino acids of the insulin molecule to form the novel compounds of formula I rather than by prolongation of the B-chain beyond the 30 residues of the mammalian insulins relates to ease in preparation. In the semisynthetic transpeptidation, a large molar excess of the amino acid amide or amino acid ester is employed. If a dipeptide amide or ester were to be used in the transpeptidation reaction, either price or solubility or both are prohibitive for use in large excess, and consequently the yield of the product becomes lower. Even when the same equimolar excess of, for example, Lys(Boc)-$NH_2$ or Lys(Boc)-Lys(Boc)-$NH_2$ is used in the transpeptidation reaction under similar conditions, the yield with the amino acid amide becomes substantially higher than with the dipeptide amide.

Insulin preparations of this invention are prepared by dissolving a compound of formula I in an aqueous medium at slightly acidic conditions, for example, in a concentration of 240 or 600 nmole/ml. The aqueous medium is made isotonic, for example, with sodium chloride, sodium acetate or glycerol. Furthermore, the aqueous medium may contain zinc ions in a concentrations of up to about 30 $\mu$g of $Zn^{++}$ per nmol of compound of formula I, buffers such as acetate, citrate and histidine and preservatives such as m-cresol, nipagin or phenol. The pH value of the final insulin preparation depends upon the number of charges that have been changed in the compound of formula I, the concentration of zinc ions, the concentration of the compound of formula I and the compound of formula I selected. The pH value is adjusted to a value convenient for administration such as about 2.5 - 4.5, preventing precipitation. The insulin preparation is made sterile by sterile filtration.

The insulin preparations of this invention are used similarly to the use of the known insulin preparations.

Any novel feature or combination of features described herein is considered essential to this invention.

Herein the abbreviations used for the amino acids are those stated in J.Biol.Chem. 243 (1968), 3558. The amino acids stated herein are in L configuration. In formula I and elsewhere herein, A(1-3) is Gly-Ile-Val, A(5-6) is Gln-Cys etc., cf. the amino acid sequence of human insulin. Unless otherwise indicated, the species of insulins stated herein is human.

Synthesis of the insulin compounds

The source of insulin was an insulin precursor expressed in yeast as described in European patent application publication No. 163.529.

The insulin precursors were recovered from the fermentation broths by adsorption to LiChroprep™ RP-18 as described in Example 7 of the same European patent application. The precursors were eluted from the column with 0.2 M KCl, 0.001 M HCl in 33% (v/v) ethanol. The insulin precursors were crystallized from the pool by successive additions of water (1 volume per volume of pool), solid trisodium citrate to obtain a molarity of 0.05 M and finally zinc acetate to obtain a molarity of 0.006 M. The pH value was adjusted to 6.8 and the mixture was left overnight at 4°C. The crystals were isolated by centrifugaton, washed with water

and dried in vacuo.

Protected amino acids and protected peptides for enzymatic semisynthesis were either prepared by standard methods or purchased (custom synthesis) from either Nova Biochem or Bachem, both Switzerland.

The letters ™ after a name indicates that it is a trade mark.

In the starting material in Examples 1 through 14, $(Q_q\text{-}T)_r$ of formula II was chosen to Ala-Ala-Lys and constructed as described for yeast plasmid pMT610 in Example 10 in European patent application publication No. 163.529. Nucleotides coding for $Gln^{B13}$, $Gln^{A17}$, $Arg^{B27}$, $Lys^{B27}$, $Asp^{A21}$, $Gly^{A21}$, $His^{A21}$, $Ser^{A21}$ and $Thr^{A21}$ were substituted in pMT610 by site specific mutagenisis using the procedure in Nucl.Acids.Res. 11 (1983), 5103 - 5112.

## Example 1

### Synthesis of $Gln^{13}$,$Arg^{B27}$ human insulin

To a suspension of 5 g of $Gln^{B13}$,$Arg^{B27}$,B(1-29)-Ala-Ala-Lys-A(1-21) insulin precursor in 50 ml of 2 M $Thr\text{-}OBu^t$,$CH_3$ COOH (L-threonine tert.butyl ester, hydroacetate salt) in DMF, 25 ml of 25.5% (v/v) water in DMF (25.5 ml water, DMF to make 100 ml) was added. The suspension was cooled to 12 °C under stirring. A solution of 0.5 g of porcine trypsin in 12.5 ml of a 0.05 M aqueous solution of calcium acetate was added. Stirring was continued until dissolution. After 48 hours at 12 °C, the proteins were precipitated by pouring the mixture into 600 ml of acetone. The precipitate was isolated by centrifugaton, washed once with 200 ml of acetone, isolated by centrifugation and dried in a stream of nitrogen. The precipitate was dissolved in 100 ml of 0.04 N hydrochloric acid, the pH value was adjusted to 2.5 and the solution was applied to a 5 x 30 cm preparative high pressure liquid chromatography (hereinafter designated HPLC) column packed with silica particles substituted with octadecyldimethylsilyl (mean particle size 15 micron, pore size 100 Ångstrøm). The column was equilibrated with ethanol/0.3 M aqueous solution of potassium chloride, 0.001 N hydrochloric acid, in a ratio of 35.5/64.5 (parts per volume). The proteins were eluted from the column with the same buffer at a rate of 2 litre/h.

$Gln^{B13}$,$Arg^{B27}$,$Thr^{B30}$-$OBu^t$ human insulin was found in a peak emerging from the column between 55 and 100 min. The $Gln^{B13}$,$Arg^{B27}$,$Thr^{B30}$-$OBu^t$ human insulin was isolated from the pool by successive additions of water to make ethanol concentration 15% (v/v), solid trisodium citrate to obtain a molarity of 0.05 M with respect to citrate and solid zinc chloride to obtain a molarity of 0.006 M with respect to zinc. The pH value was adjusted to 6.8 and after 1 hour at room temperature, the crystallisation was continued at 4 °C for 24 hours with stirring. The crystals were spun down, washed twice with 20 ml of ice-cold water, spun down and dried in vacuo. Yield: 2.51 g of $Gln^{B13}$,$Arg^{B27}$,$Thr^{B30}$-$OBu^t$ human insulin.

$Gln^{B13}$,$Arg^{B27}$,$Thr^{B30}$-$OBu^t$ human insulin was dissolved in 100 ml of trifluoroacetic acid and left for 2 hours at room temperature. The trifluoroacetic acid was removed by lyophilization. The lyophilisate was dissolved in 100 ml of water, the pH value adjusted to 2.5 and 20 g of sodium chloride was added. The salt cake consisting of $Gln^{B13}$,$Arg^{B27}$ human insulin was isolated by centrifugation. The salt cake was dissolved in 850 ml of water and $Gln^{B13}$,$Arg^{B27}$ human insulin was crystallized by successive additions of 150 ml of ethanol, 14.7 g of trisodium citrate,dihydrate and 0.82 g of zinc chloride followed by adjustment of the pH value to 6.8. After 1 hour at room temperature, the crystallisation was continued at 4 °C for 24 hours with gentle stirring. The crystals were spun down, washed twice with 20 ml of ice-cold water, spun down and dried in vacuo. Yield: 1.71 g of $Gln^{B13}$,$Arg^{B27}$ human insulin, corresponding to 36%.

The amino acid composition was in agreement with the theory, arginine being 2 residues/molecule. The product was pure in DISC PAGE electrophoresis, the rate of migration being 55% of that of human insulin corresponding to a difference in charges of about 2. For details of the DISC PAGE electrophoresis see Horm.Metab.Res. Supplement Series No. 5 (1974), 134. The content of zinc in the crystals was 0.42% (weight/weight).

## Examples 2 - 6

### Synthesis of $Gln^{B13}$,$Gln^{A17}$ human insulin, $Gln^{A17}$,$Arg^{B27}$ human insulin, $Arg^{B27}$ human insulin, $Lys^{B27}$ human insulin and $His^{A21}$,$Arg^{B27}$ human insulin

The 5 title compounds were synthesized from the corresponding single chain insulin precursors, viz.

$Gln^{B13}$,$Gln^{A17}$,B(1-29)-Ala-Ala-Lys-A(1-21),

$Gln^{A17}$,$Arg^{B27}$,B(1-29)-Ala-Ala-Lys-A(1-21),

$Arg^{B27}$,B(1-29)-Ala-Ala-Lys-A(1-21),
$Lys^{B27}$,B(1-29)-Ala-Ala-Lys-A(1-21) and
$His^{A21}$,$Arg^{B27}$,B(1-29)-Ala-Ala-Lys-A(1-21), using the methods described in Example 1. Yields, charges relative to human insulin, rates of migration relative to insulin in DISC PAGE electrophoresis at pH 8.9 and deviations in amino acid compositions from human insulin appear from Table I, below.

Examples 7 - 13

Synthesis of $Asp^{A21}$,$Arg^{B27}$,$Thr^{B30}$-$NH_2$ human insulin, $Gly^{A21}$,$Arg^{B27}$,$Thr^{B30}$-$NH_2$ human insulin, $His^{A21}$,$Arg^{B27}$,$Thr^{B30}$-$NH_2$ human insulin, $Gly^{A21}$,$Arg^{B27}$,$Gln^{B13}$,$Thr^{B30}$-$NH_2$ human insulin, $Ser^{A21}$,$Arg^{B27}$,$Gln^{B13}$,$Thr^{B30}$-$NH_2$ human insulin, $Thr^{A21}$,$Arg^{B27}$,$Gln^{B13}$,$Thr^{B30}$-$NH_2$ human insulin and $Ser^{A21}$,$Arg^{B27}$,$Thr^{B30}$-$NH_2$ human insulin

The 7 title compounds were synthesized from the corresponding single chain insulin precursors, viz.
$Asp^{A21}$,$Arg^{B27}$,B(1-29)-Ala-Ala-Lys-A(1-21),
$Gly^{A21}$,$Arg^{B27}$,B(1-29)-Ala-Ala-Lys-A(1-21),
$His^{A21}$,$Arg^{B27}$,B(1-29)-Ala-Ala-Lys-A(1-21),
$Gly^{A21}$,$Arg^{B27}$,$Gln^{B13}$,B(1-29)-Ala-Ala-Lys-A(1-21),
$Ser^{A21}$,$Arg^{B27}$,$Gln^{B13}$,B(1-29)-Ala-Ala-Lys-A(1-21),
$Thr^{A21}$,$Arg^{B27}$,$Gln^{B13}$,B(1-29)-Ala-Ala-Lys-A(1-21) and
$Ser^{A21}$,$Arg^{B27}$,B(1-29)-Ala-Ala-Lys-A(1-21) by tryptic transpeptidation in organic aqueous solution in the presence of Thr-$NH_2$ as described in European patent application publication No. 194.864, Examples 4 and 6. Yields, charges relative to human insulin, rates of migration relative to insulin in DISC PAGE electrophoresis at pH 8.9 and deviations in amino acid compositions from human insulin appear from Table I.

Example 14

Synthesis of $Asp^{A21}$,$Arg^{B27}$,$Lys^{B30}$-$NH_2$ human insulin

The title compound was synthesized from the corresponding single chain insulin precursor, viz. $Asp^{A21}$,$Arg^{B27}$,B(1-29)-Ala-Ala-Lys-A(1-21) by tryptic transpeptidation in organic aqueous solution in the presence of Lys(Boc)-$NH_2$, purification of the intermediate, $Lys(Boc)^{B30}$-$NH_2$ human insulin, followed by removal of the Boc protecting group by TFA as described in European patent application publication No. 194.864, Examples 5 and 7. Yield and analytical data are shown in Table I.

Table I

| Substitution in human insulin | Yield, % | Charge relative to human insulin at pH 7 | Rate of migration at pH 8.9, % relative to human insulin | Deviations in amino acid compositions from human insulin after acid hydrolysis, residues/molecule |
|---|---|---|---|---|
| $Gln^{B13}, Gln^{A17}$ | 46 | +2 | 55 | none |
| $Gln^{A17}, Arg^{B27}$ | 32 | +2 | 55 | +1 Arg, -1 Thr |
| $Arg^{B27}$ | 35 | +1 | 75 | +1 Arg, -1 Thr |
| $Lys^{B27}$ | 39 | +1 | 75 | +1 Lys, -1 Thr |
| $His^{A21}, Arg^{B27}$ | 16 | +1.1 | 75 | +1 His, +1 Arg, -1 Asp, -1 Thr |
| $Asp^{A21}, Arg^{B27}, Thr^{B30}-NH_2$ | 23 | +1 | 75 | +1 Arg, -1 Thr |
| $Gly^{A21}, Arg^{B27}, Thr^{B30}-NH_2$ | 32 | +2 | 55 | +1 Gly, +1 Arg, -1 Asp, -1 Thr |
| $His^{A21}, Arg^{B27}, Thr^{B30}-NH_2$ | 20 | +2.1 | 55 | +1 His, +1 Arg, -1 Asp, -1 Thr |
| $Gly^{A21}, Arg^{B27}, Gln^{B13}, Thr^{B30}-NH_2$ | 23 | +3 | 35 | +1 Gly, +1 Arg, -1 Asp, -1 Thr |
| $Ser^{A21}, Arg^{B27}, Gln^{B13}, Thr^{B30}-NH_2$ | 21 | +3 | 35 | +1 Ser, +1 Arg, -1 Asp, -1 Thr |
| $Thr^{A21}, Arg^{B27}, Gln^{B13}, Thr^{B30}-NH_2$ | 29 | +3 | 35 | +1 Arg, -1 Asp |
| $Ser^{A21}, Arg^{B27}, Thr^{B30}-NH_2$ | | +2 | 55 | +1 Ser, +1 Arg, -1 Asp, -1 Thr |
| $Asp^{A21}, Arg^{B27}, Lys^{B30}-NH_2$ | 13 | +2 | 55 | +1 Arg, +1 Lys, -2 Thr |

Example 15

Preparation of injectable solutions of compounds of formula I

Sterile injectable solutions of the compounds of formula I for testing of the degree of prolonged action were made using 1.6% (w/v) glycerol as the isotonicum, using 0.3% (w/v) m-cresol as the preservative, and being buffered with 0.01 M sodium acetate. The concentration of zinc ions was 8 or 80 µg/ml. The pH values of the solutions were adjusted sufficiently off the isoelectric point of the compounds of formula I to keep the solutions clear upon storage at 4°C. The solutions contained 240 nmole/ml of the compounds of formula I. The concentration of 240 nmole/ml was established by measurement of the absorbance at 276 nm of a more concentrated stock solution devoid of m-cresol, using the molar extinction coefficient for porcine insulin of 6100 for these derivatives (see Handbuch der Inneren Medizin, Vol. 7/Part 2A, Editor: Oberdisse, 1975, 113). For monocomponent procine insulin, the established potency is 28.5 U/mg dry substance (see Diabetes Care, Vol. 6/Supplement 1 (1983), 4), viz. 1 U corresponds to 5.95 nmole.

Injectable solutions containing 240 nmole/ml of the compounds of formula I stated in Table II and having the pH values and content of zinc stated therein were made.

Test for prolongation of insulin effect

The prolongation of the hypoglycemic effect produced by the injectable solutions of insulin was tested according to British Pharmacopoeia 1980, A 142, in fasted rabbits. Each test solution was administered subcutaneously in a dosis of 14.3 nmole per rabbit in 12 animals weighing 3 - 4 kg, and the course of the hypoglycemia was followed for 6 hours. For comparison the fast acting preparation, Actrapid™ porcine insulin and the intermediate acting Monotard™ human insulin, were included in the tests. The results of the tests are shown in Table II.

Table II

| Compound of formula I | $Zn^{++}$, µg/ml | pH | Glucose in percent of initial | | | |
|---|---|---|---|---|---|---|
| | | | 1 h | 2 h | 4 h | 6 h |
| $Gln^{A17}$,$Arg^{B27}$ human insulin | 80 | 4.5 | 53 | 47 | 50 | 66 |
| $Gln^{B13}$,$Arg^{B27}$ human insulin | 80 | 4.5 | 55 | 46 | 61 | 91 |
| $Gln^{A17}$,$Gln^{B13}$ human insulin | 80 | 4.5 | 53 | 47 | 55 | 82 |
| $Arg^{B27}$ human insulin | 80 | 4.5 | 45 | 34 | 51 | 91 |
| $Lys^{B27}$ human insulin | 80 | 4.5 | 47 | 40 | 55 | 93 |
| $Asp^{A21}$,$Arg^{B27}$,$Thr^{B30}$-$NH_2$ human insulin | 80 | 4 | 60 | 54 | 58 | 60 |
| $Asp^{A21}$,$Arg^{B27}$,$Lys^{B30}$-$NH_2$ human insulin | 80 | 4 | 72 | 67 | 61 | 59 |
| $Gly^{A21}$,$Arg^{B27}$,$Thr^{B30}$-$NH_2$ human insulin | 8 | 4 | 59 | 62 | 71 | 74 |
| $Gly^{A21}$,$Arg^{B27}$,$Thr^{B30}$-$NH_2$ human insulin | 80 | 4 | 72 | 73 | 74 | 74 |
| $His^{A21}$,$Arg^{B27}$ human insulin | 80 | 4 | 65 | 53 | 66 | 88 |
| $His^{A21}$,$Arg^{B27}$,$Thr^{B30}$-$NH_2$ human insulin | 80 | 4 | 61 | 52 | 52 | 72 |
| $Gly^{A21}$,$Arg^{B27}$,$Gln^{B13}$,$Thr^{B30}$-$NH_2$ human insulin | 80 | 4 | 82 | 86 | 85 | 90 |
| $Ser^{A21}$,$Arg^{B27}$,$Gln^{B13}$,$Thr^{B30}$-$NH_2$ human insulin | 80 | 4 | 90 | 91 | 88 | 92 |
| $Thr^{A21}$,$Arg^{B27}$,$Gln^{B13}$,$Thr^{B30}$-$NH_2$ human insulin | 80 | 4 | 90 | 90 | 88 | 93 |
| $Ser^{A21}$,$Arg^{B27}$,$Thr^{B30}$-$NH_2$ human insulin | 80 | 4 | 60 | 62 | 64 | 68 |
| Actrapid™ porcine insulin | 15 | 7 | 46 | 44 | 74 | 91 |
| Monotard™ human insulin | 80 | 7 | 54 | 43 | 50 | 74 |

The potencies of insulin compounds were assessed in the mouse blood sugar depletion test (British Pharmacopoeia 1980, A 141 - A 142). In order to minimize the problem of estimating potency of insulins having a timing different from the standard, insulin solutions for potency determinations were made up without additions of zinc. Solutions were made up to contain 240 nmole/ml based on the absorbance at 276 nm. The zinc content of solutions were 8 - 10 µg/ml, arizing from the crystalline derivatives. The estimated potencies of some insulin compounds are shown in Table III, below.

Table III

| | Potency relative to insulin, % | Confidence limits (P = 0.05), % |
|---|---|---|
| $Gln^{A17}$,$Arg^{B27}$ human insulin | 69 | 79 - 62 |
| $Gln^{B13}$,$Arg^{B27}$ human insulin | 78 | 88 - 69 |
| $Gln^{B13}$,$Gln^{A17}$ human insulin | 50 | 63 - 40 |
| $Arg^{B27}$ human insulin | 87 | 95 - 80 |
| $Lys^{B27}$ human insulin | 88 | 97 - 80 |
| $Asp^{A21}$,$Arg^{B27}$,$Thr^{B30}$-$NH_2$ human insulin | 83 | 92 - 74 |
| $Asp^{A21}$,$Arg^{B27}$,$Lys^{B30}$-$NH_2$ human insulin | 69 | 77 - 62 |
| $Gly^{A21}$,$Arg^{B27}$,$Thr^{B30}$-$NH_2$ human insulin | 75 | 83 - 68 |
| $His^{A21}$,$Arg^{B27}$ human insulin | 71 | 79 - 63 |
| $His^{A21}$,$Arg^{B27}$,$Thr^{B30}$-$NH_2$ human insulin | 72 | 81 - 64 |
| $Gly^{A21}$,$Arg^{B27}$,$Gln^{B13}$,$Thr^{B30}$-$NH_2$ human insulin | 49 | 54 - 44 |
| $Ser^{A21}$,$Arg^{B27}$,$Gln^{B13}$,$Thr^{B30}$-$NH_2$ human insulin | 47 | 54 - 40 |
| $Thr^{A21}$,$Arg^{B27}$,$Gln^{B13}$,$Thr^{B30}$-$NH_2$ human insulin | 28 | 32 - 24 |
| $Ser^{A21}$,$Arg^{B27}$,$Thr^{B30}$-$NH_2$ human insulin | 76 | 83 - 68 |

The features disclosed in the foregoing description and in the following claims may, both separately and in any combination thereof, be material for realising the invention in diverse forms thereof.

## Claims

**1.** Compounds of the general formula I

```
A(1-3)-E^1-A(5-6)-Cys-A(8-16)-E^2-A(18-19)-Cys-W          (A-chain)
                   |                          |
                   S                          S
                   |                          |
                   S                          S                (I)
                   |                          |
       B(1-6)-Cys-B(8-12)-E^3-B(14-18)-Cys
                                              |            (B-chain)
       R-Z_n-Y_m-Lys-Pro-X-B(26-22)-E^4-Gly
```

wherein the letters A and B followed by figures in parentheses designate the peptide fragments of the A- and B-chains, respectively, indicated by the figures in parentheses, $E^1$, $E^2$, $E^3$ and $E^4$ are the same or different each representing glutamic acid or a neutral amino acid residue which can be coded for by nucleotide sequences, X represents an L-threonine, L-arginine or L-lysine residue, Y and Z are the same or different and each represent an amino acid residue wherein any side chain amino group may be acylated and wherein any side chain hydroxy group may be alkylated, m and n are the same or different and each represent zero or one, and R represents hydroxy or an amido or ester residue which blocks the C-terminal carboxy group of the B-chain, and W represents an amino acid residue except asparagine, with the proviso that at least ore of the six amino acid residues $E^1$, $E^2$, $E^3$, $E^4$, W and X is different from the amino acid residues present at the corresponding positions in human insulin, and that at least one of the eight amino acid residues $E^1$, $E^2$, $E^3$, $E^4$, W, X, Y and Z and the group R are selected so that the compound of formula I has at least one charge more than human insulin at a pH value of 7.

**2.** Compound according to Claim 1, charaterized in that X is a lysine or arginine residue.

3. Compounds according to any one of the preceding claims, characterized in that $E^2$ and $E^3$ each is a glutamine residue.

4. Compounds according to any one of the preceding claims, characterized in that Y and/or Z is a basic amino acid residue wherein the side chain amino group optionally is acylated (m = 1).

5. Compounds according to any one of the preceding claims, characterized in that n is zero, and Y is a basic amino acid residue (m = 1).

6. Compounds according to any one of the preceding claims, characterized in that Y and Z are both basic amino acid residues (m = 1, n = 1).

7. Compounds according to any one of the preceding claims, characterized in that R is a group of the general formula $-NR^1R^2$ wherein $R^1$ and $R^2$ are the same or different and each represent hydrogen or lower alkyl, and preferably R is $-NH_2$.

8. Compounds according to any one of the Claims 1 through 6, characterized in that R is lower alkoxy, preferably tertiary butyloxy.

9. Compounds according to any one of the Claims 1 through 6, characterized in that R is a residue of a lactam which preferably contains less than 8 atoms in the lactam ring.

10. Compounds according to any of the preceding claims, characterized in that W is Gly, Ser, Thr, Ala, His, Asp or hSer.

11. Compound according to Claim 1, characterized in that it is
$Gly^{A21}$,$Lys^{B27}$,$Thr^{B30}$-$NH_2$ human insulin,
$Ser^{A21}$,$Lys^{B27}$,$Thr^{B30}$-$NH_2$ human insulin,
$Thr^{A21}$,$Lys^{B27}$,$Thr^{B30}$-$NH_2$ human insulin,
$Ala^{A21}$,$Lys^{B27}$,$Thr^{B30}$-$NH_2$ human insulin,
$His^{A21}$,$Lys^{B27}$,$Thr^{B30}$-$NH_2$ human insulin,
$Asp^{A21}$,$Lys^{B27}$,$Thr^{B30}$-$NH_2$ human insulin,
$Gly^{A21}$,$Arg^{B27}$,$Thr^{B30}$-$NH_2$ human insulin,
$Ser^{A21}$,$Arg^{B27}$,$Thr^{B30}$-$NH_2$ human insulin,
$Thr^{A21}$,$Arg^{B27}$,$Thr^{B30}$-$NH_2$ human insulin,
$Ala^{A21}$,$Arg^{B27}$,$Thr^{B30}$-$NH_2$ human insulin,
$His^{A21}$,$Arg^{B27}$,$Thr^{B30}$-$NH_2$ human insulin,
$Asp^{A21}$,$Arg^{B27}$,$Thr^{B30}$-$NH_2$ human insulin,
$Gln^{B13}$,$Gly^{A21}$,$Arg^{B27}$,$Thr^{B30}$-$NH_2$ human insulin,
$Gln^{B13}$,$Ser^{A21}$,$Arg^{B27}$,$Thr^{B30}$-$NH_2$ human insulin,
$Gln^{B13}$,$Thr^{A21}$,$Arg^{B27}$,$Thr^{B30}$-$NH_2$ human insulin,
$Gln^{B13}$,$Ala^{A21}$,$Arg^{B27}$,$Thr^{B30}$-$NH_2$ human insulin,
$Gln^{B13}$,$His^{A21}$,$Arg^{B27}$,$Thr^{B30}$-$NH_2$ human insulin,
$Gln^{B13}$,$Asp^{A21}$,$Arg^{B27}$,$Thr^{B30}$-$NH_2$ human insulin,
$Gln^{B13}$,$Gly^{A21}$,$Lys^{B27}$,$Thr^{B30}$-$NH_2$ human insulin,
$Gln^{B13}$,$Ser^{A21}$,$Lys^{B27}$,$Thr^{B30}$-$NH_2$ human insulin,
$Gln^{B13}$,$Thr^{A21}$,$Lys^{B27}$,$Thr^{B30}$-$NH_2$ human insulin,
$Gln^{B13}$,$Ala^{A21}$,$Lys^{B27}$,$Thr^{B30}$-$NH_2$ human insulin,
$Gln^{B13}$,$His^{A21}$,$Lys^{B27}$,$Thr^{B30}$-$NH_2$ human insulin,
$Gln^{B13}$,$Asp^{A21}$,$Lys^{B27}$,$Thr^{B30}$-$NH_2$ human insulin,
$Ser^{A21}$,$Lys^{B27}$ human insulin,
$Thr^{A21}$,$Lys^{B27}$ human insulin,
$Ala^{A21}$,$Lys^{B27}$ human insulin,
$His^{A21}$,$Lys^{B27}$ human insulin,
$Asp^{A21}$,$Lys^{B27}$ human insulin,
$Gly^{A21}$,$Lys^{B27}$ human insulin,
$Ser^{A21}$,$Arg^{B27}$ human insulin,
$Thr^{A21}$,$Arg^{B27}$ human insulin,

$Ala^{A21}$,$Arg^{B27}$ human insulin,
$His^{A21}$,$Arg^{B27}$ human insulin,
$Asp^{A21}$,$Arg^{B27}$,$Lys^{B30}$-$NH_2$ human insulin
$Asp^{A21}$,$Arg^{B27}$ human insulin,
$Gly^{A21}$,$Arg^{B27}$ human insulin,
$Gln^{A17}$,$Ser^{A21}$,$Arg^{B27}$ human insulin,
$Gln^{A17}$,$Thr^{A21}$,$Arg^{B27}$ human insulin,
$Gln^{A17}$,$Ala^{A21}$,$Arg^{B27}$ human insulin,
$Gln^{A17}$,$His^{A21}$,$Arg^{B27}$ human insulin,
$Gln^{A17}$,$Asp^{A21}$,$Arg^{B27}$ human insulin,
$Gln^{A17}$,$Gly^{A21}$,$Arg^{B27}$ human insulin,
$Gln^{A17}$,$Ser^{A21}$,$Gln^{B13}$ human insulin,
$Gln^{A17}$,$Thr^{A21}$,$Gln^{B13}$ human insulin,
$Gln^{A17}$,$Ala^{A21}$,$Gln^{B13}$ human insulin,
$Gln^{A17}$,$His^{A21}$,$Gln^{B13}$ human insulin,
$Gln^{A17}$,$Asp^{A21}$,$Gln^{B13}$ human insulin,
$Gln^{A17}$,$Gly^{A21}$,$Gln^{B13}$ human insulin,
$Arg^{B27}$,$Ser^{A21}$,$Gln^{B13}$ human insulin,
$Arg^{B27}$,$Thr^{A21}$,$Gln^{B13}$ human insulin,
$Arg^{B27}$,$Ala^{A21}$,$Gln^{B13}$ human insulin,
$Arg^{B27}$,$His^{A21}$,$Gln^{B13}$ human insulin,
$Arg^{B27}$,$Asp^{A21}$,$Gln^{B13}$ human insulin,
$Arg^{B27}$,$Gly^{A21}$,$Gln^{B13}$ human insulin,
$Gln^{A17}$,$Ser^{A21}$,$Lys^{B27}$ human insulin,
$Gln^{A17}$,$Thr^{A21}$,$Lys^{B27}$ human insulin,
$Gln^{A17}$,$Ala^{A21}$,$Lys^{B27}$ human insulin,
$Gln^{A17}$,$His^{A21}$,$Lys^{B27}$ human insulin,
$Gln^{A17}$,$Asp^{A21}$,$Lys^{B27}$ human insulin,
$Gln^{A17}$,$Gly^{A21}$,$Lys^{B27}$ human insulin,
$Gln^{B13}$,$Ser^{A21}$,$Lys^{B27}$ human insulin,
$Gln^{B13}$,$Thr^{A21}$,$Lys^{B27}$ human insulin,
$Gln^{B13}$,$Ala^{A21}$,$Lys^{B27}$ human insulin,
$Gln^{B13}$,$His^{A21}$,$Lys^{B27}$ human insulin,
$Gln^{B13}$,$Asp^{A21}$,$Lys^{B27}$ human insulin,
$Gln^{B13}$,$Gly^{A21}$,$Lys^{B27}$ human insulin or
$hSer^{A21}$, $Arg^{B27}$, $Thr^{B30}$-$NH_2$ human insulin.

**12.** Injectable solutions with prolonged insulin action, characterized in that they contain a compound of the general formula I

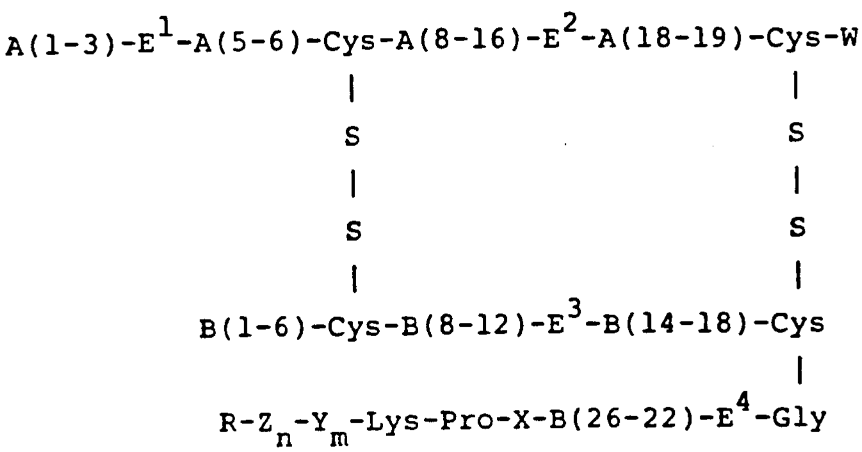

(A-chain)

(I)

(B-chain)

wherein the letters A and B followed by figures in parentheses designate the peptide fragments of the A- and B-chains, respectively, indicated by the figures in parentheses, $E^1$, $E^2$, $E^3$ and $E^4$ are the same

or different each representing glutamic acid or a neutral amino acid residue which can be coded for by nucleotide sequences, X represents an L-threonine, L-arginine or L-lysine residue, Y and Z are the same or different and each represent an amino acid residue wherein any side chain amino group may be acylated and wherein any side chain hydroxy group may be alkylated, m and n are the same or different and each represent zero or one, and R represents hydroxy or an amido or ester residue which blocks the C-terminal carboxyl group of the B-chain, and W represents an amino acid residue except aspargine; with the proviso that at least one of the six amino acid residues $E^1$, $E^2$, $E^3$, $E^4$, W and X is different from the amino acid residues present at the corresponding positions in human insulin, and that at least one of the eight amino acid residues $E^1$, $E^2$, $E^3$, $E^4$, W, X, Y and Z and the group R are selected so that the compound of formula I has at least one charge more than human insulin at a pH value of 7.

**13.** Preparation according to Claim 12, characterized in that it contains zinc ions, preferably from about 2 µg to about 2 mg zinc per ml, most prefered from about 5 µg to 200 µg zinc per ml.

**14.** Zinc solution for use in preparing a more prolonged acting solution according to Claim 12 by mixing it with a solution containing a compound of formula I stated in Claim 11, which zinc solution preferably contains between about 10 µg and 20 mg zinc per ml.

**15.** A method for preparing a solution according to Claim 12, characterized in that a solution of a compound of formula I stated in Claim 12, optionally containing zinc, is mixed with a zinc solution optionally containing a compound of formula I stated in Claim 12, rendering a mixture with a zinc concentration of up to 2 mg/ml, and preferably the content of zinc in each of the two solutions is less than about 4 mg/ml.

**16.** A process for preparing compounds of the general formula I stated in Claim 12, characterized in transpeptidating an insulin precurser of the general formula II:

$$\begin{array}{l} X\text{-}B(26\text{-}22)\text{-}E^4\text{-}B(20\text{-}14)\text{-}E^3\text{-}B(12\text{-}1) \\ | \\ B(28\text{-}29)\text{-}(Q_q\text{-}T)_r\text{-}A(1\text{-}3)\text{-}E^1\text{-}A(5\text{-}16)\text{-}E^2\text{-}A(18\text{-}20)\text{-}W \end{array} \quad (II)$$

wherein A and B designate the fragments of the A- and B-chains indicated by numbers in parentheses, Q is a peptide chain with q amino acids, q is an integer from 0 to 33, T is Lys or Arg, and r is zero or one and $E^1$, $E^2$, $E^3$, $E^4$, W and X each are as defined in Claim 12, with a compound of the general formula III:

$H\text{-}Y_m\text{-}Z_n\text{-}R$ (III)

where Y, Z, R, m and n each are as defined above, and wherein side chain amino groups and hydroxy groups in Y and Z optionally are blocked with amino and hydroxy protecting groups, using trypsin or a trypsin like enzyme as a catalyst.

## Patentansprüche

1. Verbindungen der allgemeinen Formel I

```
A(1-3)-E¹-A(5-6)-Cys-A(8-16)-E²-A(18-19)-Cys-W          (A-Kette)
                   |                         |
                   S                         S
                   |                         |                    (I)
                   S                         S
                   |                         |
          B(1-6)-Cys-B(8-12)-E³-B(14-18)-Cys
                                             |              (B-Kette)
          R-Zn-Ym-Lys-Pro-X-B(26-22)-E⁴-Gly,
```

wobei die Buchstaben A und B, gefolgt von Zahlen in Klammern, die Peptidfragmente der A- bzw. B-Ketten bezeichnen, angegeben durch die Zahlen in Klammern, $E^1$, $E^2$, $E^3$ und $E^4$ identisch oder verschieden sind, wobei jedes Glutaminsäure oder einen neutralen Aminosäurerest darstellt, der durch Nucleotidsequenzen kodiert werden kann, X einen L-Threonin-, L-Arginin- oder L-Lysin-Rest darstellt, Y und Z identisch oder verschieden sind und jedes einen Aminosäurerest darstellt, in dem jede Seitenkettenaminogruppe acyliert sein kann und in dem jede Seitenkettenhydroxygruppe alkyliert sein kann, m und n identisch oder verschieden sind und jedes Null oder Eins darstellt und R Hydroxy oder einen Amino- oder Esterrest darstellt, der die C-terminale Carboxylgruppe der B-Kette blockiert, und W einen Aminosäurerest, ausgenommen Asparagin, darstellt, mit der Maßgabe, daß wenigstens einer der sechs Aminosäurereste $E^1$, $E^2$, $E^3$, $E^4$, W und X von den in den entsprechenden Positionen in Human-Insulin vorliegenden Aminosäureresten verschieden ist und daß wenigstens einer der acht Aminosäurereste $E^1$, $E^2$, $E^3$, $E^4$, W, X, Y und Z und die Gruppe R so ausgewählt sind, daß die Verbindung von Formel I wenigstens eine Ladung mehr als Human-Insulin bei einem pH-Wert von 7 besitzt.

2. Verbindung nach Anspruch 1, dadurch gekennzeichnet, daß X ein Lysin- oder Arginin-Rest ist.

3. Verbindungen nach einem der vorangehenden Ansprüchen, dadurch gekennzeichnet, daß $E^2$ und $E^3$ jeweils ein Glutamin-Rest ist.

4. Verbindungen nach einem der vorangehenden Ansprüche, dadurch gekennzeichnet, daß Y und/oder Z ein basischer Aminosäurerest ist, in dem die Seitenkettenaminogruppe fakultativ acyliert ist (m = 1).

5. Verbindungen nach einem der vorangehenden Ansprüche, dadurch gekennzeichnet, daß n Null ist und Y ein basischer Aminosäurerest ist (m = 1).

6. Verbindungen nach einem der vorangehenden Ansprüche, dadurch gekennzeichnet, daß Y und Z beide basische Aminosäurereste sind (m = 1, n = 1).

7. Verbindungen nach einem der vorangehenden Ansprüche, dadurch gekennzeichnet, daß R eine Gruppe der allgemeinen Formel $-NR^1R^2$ ist, wobei $R^1$ und $R^2$ identisch oder verschieden sind und jedes Wasserstoff und Niederalkyl darstellt und R vorzugsweise $-NH_2$ ist.

8. Verbindungen nach einem der Ansprüche 1 bis 6, dadurch gekennzeichnet, daß R Niederalkoxy, vorzugsweise tertiäres Butyloxy ist.

9. Verbindungen nach einem der Ansprüche 1 bis 6, dadurch gekennzeichnet, daß R ein Rest eines Lactams ist, der vorzugsweise weniger als 8 Atome in Lactamring enthält.

**10.** Verbindungen nach einem der vorangehenden Ansprüche, dadurch gekennzeichnet, daß W Gly, Ser, Thr, Ala, His, Asp oder hSer ist.

**11.** Verbindungen nach Anspruch 1, dadurch gekennzeichnet, daß sie
$Gly^{A21}$,$Lys^{B27}$,$Thr^{B30}$-$NH_2$-Human-Insulin,
$Ser^{A21}$,$Lys^{B27}$,$Thr^{B30}$-$NH_2$-Human-Insulin,
$Thr^{A21}$,$Lys^{B27}$,$Thr^{B30}$-$NH_2$-Human-Insulin,
$Ala^{A21}$,$Lys^{B27}$,$Thr^{B30}$-$NH_2$-Human-Insulin,
$His^{A21}$,$Lys^{B27}$,$Thr^{B30}$-$NH_2$-Human-Insulin,
$Asp^{A21}$,$Lys^{B27}$,$Thr^{B30}$-$NH_2$-Human-Insulin,
$Gly^{A21}$,$Arg^{B27}$,$Thr^{B30}$-$NH_2$-Human-Insulin,
$Ser^{A21}$,$Arg^{B27}$,$Thr^{B30}$-$NH_2$-Human-Insulin,
$Thr^{A21}$,$Arg^{B27}$,$Thr^{B30}$-$NH_2$-Human-Insulin,
$Ala^{A21}$,$Arg^{B27}$,$Thr^{B30}$-$NH_2$-Human-Insulin,
$His^{A21}$,$Arg^{B27}$,$Thr^{B30}$-$NH_2$-Human-Insulin,
$Asp^{A21}$,$Arg^{B27}$,$Thr^{B30}$-$NH_2$-Human-Insulin,
$Gln^{B13}$,$Gly^{A21}$,$Arg^{B27}$,$Thr^{B30}$-$NH_2$-Human-Insulin,
$Gln^{B13}$,$Ser^{A21}$,$Arg^{B27}$,$Thr^{B30}$-$NH_2$-Human-Insulin,
$Gln^{B13}$,$Thr^{A21}$,$Arg^{B27}$,$Thr^{B30}$-$NH_2$-Human-Insulin,
$Gln^{B13}$,$Ala^{A21}$,$Arg^{B27}$,$Thr^{B30}$-$NH_2$-Human-Insulin,
$Gln^{B13}$,$His^{A21}$,$Arg^{B27}$,$Thr^{B30}$-$NH_2$-Human-Insulin,
$Gln^{B13}$,$Asp^{A21}$,$Arg^{B27}$,$Thr^{B30}$-$NH_2$-Human-Insulin,
$Gln^{B13}$,$Gly^{A21}$,$Lys^{B27}$,$Thr^{B30}$-$NH_2$-Human-Insulin,
$Gln^{B13}$,$Ser^{A21}$,$Lys^{B27}$,$Thr^{B30}$-$NH_2$-Human-Insulin,
$Gln^{B13}$,$Thr^{A21}$,$Lys^{B27}$,$Thr^{B30}$-$NH_2$-Human-Insulin,
$Gln^{B13}$,$Ala^{A21}$,$Lys^{B27}$,$Thr^{B30}$-$NH_2$-Human-Insulin,
$Gln^{B13}$,$His^{A21}$,$Lys^{B27}$,$Thr^{B30}$-$NH_2$-Human-Insulin,
$Gln^{B13}$,$Asp^{A21}$,$Lys^{B27}$,$Thr^{B30}$-$NH_2$-Human-Insulin,
$Ser^{A21}$,$Lys^{B27}$-Human-Insulin,
$Thr^{A21}$,$Lys^{B27}$-Human-Insulin,
$Ala^{A21}$,$Lys^{B27}$-Human-Insulin,
$His^{A21}$,$Lys^{B27}$-Human-Insulin,
$Asp^{A21}$,$Lys^{B27}$-Human-Insulin,
$Gly^{A21}$,$Lys^{B27}$-Human-Insulin,
$Ser^{A21}$,$Arg^{B27}$-Human-Insulin,
$Thr^{A21}$,$Arg^{B27}$-Human-Insulin,
$Ala^{A21}$,$Arg^{B27}$-Human-Insulin,
$His^{A21}$,$Arg^{B27}$-Human-Insulin,
$Asp^{A21}$,$Arg^{B27}$,$Lys^{B30}$-$NH_2$-Human-Insulin,
$Asp^{A21}$,$Arg^{B27}$-Human-Insulin,
$Gly^{A21}$,$Arg^{B27}$-Human-Insulin,
$Gln^{A17}$,$Ser^{A21}$,$Arg^{B27}$-Human-Insulin,
$Gln^{A17}$,$Thr^{A21}$,$Arg^{B27}$-Human-Insulin,
$Gln^{A17}$,$Ala^{A21}$,$Arg^{B27}$-Human-Insulin,
$Gln^{A17}$,$His^{A21}$,$Arg^{B27}$-Human-Insulin,
$Gln^{A17}$,$Asp^{A21}$,$Arg^{B27}$-Human-Insulin,
$Gln^{A17}$,$Gly^{A21}$,$Arg^{B27}$-Human-Insulin,
$Gln^{A17}$,$Ser^{A21}$,$Gln^{B13}$-Human-Insulin,
$Gln^{A17}$,$Thr^{A21}$,$Gln^{B13}$-Human-Insulin,
$Gln^{A17}$,$Ala^{A21}$,$Gln^{B13}$-Human-Insulin,
$Gln^{A17}$,$His^{A21}$,$Gln^{B13}$-Human-Insulin,
$Gln^{A17}$,$Asp^{A21}$,$Gln^{B13}$-Human-Insulin,
$Gln^{A17}$,$Gly^{A21}$,$Gln^{B13}$-Human-Insulin,
$Arg^{B27}$,$Ser^{A21}$,$Gln^{B13}$-Human-Insulin,
$Arg^{B27}$,$Thr^{A21}$,$Gln^{B13}$-Human-Insulin,
$Arg^{B27}$,$Ala^{A21}$,$Gln^{B13}$-Human-Insulin,
$Arg^{B27}$,$His^{A21}$,$Gln^{B13}$-Human-Insulin,
$Arg^{B27}$,$Asp^{A21}$,$Gln^{B13}$-Human-Insulin,

$Arg^{B27}$,$Gly^{A21}$,$Gln^{B13}$-Human-Insulin,
$Gln^{A17}$,$Ser^{A21}$,$Lys^{B27}$-Human-Insulin,
$Gln^{A17}$,$Thr^{A21}$,$Lys^{B27}$-Human-Insulin,
$Gln^{A17}$,$Ala^{A21}$,$Lys^{B27}$-Human-Insulin,
$Gln^{A17}$,$His^{A21}$,$Lys^{B27}$-Human-Insulin,
$Gln^{A17}$,$Asp^{A21}$,$Lys^{B27}$-Human-Insulin,
$Gln^{A17}$,$Gly^{A21}$,$Lys^{B27}$-Human-Insulin,
$Gln^{B13}$,$Ser^{A21}$,$Lys^{B27}$-Human-Insulin,
$Gln^{B13}$,$Thr^{A21}$,$Lys^{B27}$-Human-Insulin,
$Gln^{B13}$,$Ala^{A21}$,$Lys^{B27}$-Human-Insulin,
$Gln^{B13}$,$His^{A21}$,$Lys^{B27}$-Human-Insulin,
$Gln^{B13}$,$Asp^{A21}$,$Lys^{B27}$-Human-Insulin,
$Gln^{B13}$,$Gly^{A21}$,$Lys^{B27}$-Human-Insulin oder
$hSer^{A21}$,$Arg^{B27}$,$Thr^{B30}$-$NH_2$-Human-Insulin,
ist.

**12.** Injizierbare Lösungen mit verlängerter Insulinwirkung, dadurch gekennzeichnet, daß sie eine Verbindung der allgemeinen Formel I enthalten

```
A(1-3)-E¹-A(5-6)-Cys-A(8-16)-E²-A(18-19)-Cys-W          (A-Kette)
                |                           |
                S                           S
                |                           |
                S                           S                (I)
                |                           |
      B(1-6)-Cys-B(8-12)-E³-B(14-18)-Cys
                                            |               (B-Kette)
         R-Zn-Ym-Lys-Pro-X-B(26-22)-E⁴-Gly,
```

wobei die Buchstaben A und B, gefolgt von Zahlen in Klammern, die Peptidfragmente der A- bzw. B-Ketten bezeichnen, angegeben durch die Zahlen in Klammern, $E^1$, $E^2$, $E^3$ und $E^4$ identisch oder verschieden sind, wobei jedes Glutaminsäure oder einen neutralen Aminosäurerest darstellt, der durch Nucleotidsequenzen kodiert werden kann, X einen L-Threonin-, L-Arginin- oder L-Lysin-Rest darstellt, Y und Z identisch oder verschieden sind und jedes einen Aminosäurerest darstellt, in dem jede Seitenkettenaminogruppe acyliert sein kann und in dem jede Seitenkettenhydroxygruppe alkyliert sein kann, m und n identisch oder verschieden sind und jedes Null oder Eins darstellt und R Hydroxy oder einen Amido- oder Esterrest darstellt, der die C-terminale Carboxylgruppe der B-Kette blockiert, und W einen Aminosäurerest, ausgenommen Asparagin, darstellt, mit der Maßgabe, daß wenigstens einer der sechs Aminosäurereste $E^1$, $E^2$, $E^3$, $E^4$, W und X von den in den entsprechenden Positionen in Human-Insulin vorliegenden Aminosäureresten verschieden ist und daß wenigstens einer der acht Aminosäurereste $E^1$, $E^2$, $E^3$, $E^4$, W, X, Y and Z und die Gruppe R so ausgewählt sind, daß die Verbindung von Formel I wenigstens eine Ladung mehr als Human-Insulin bei einem pH-Wert von 7 besitzt.

**13.** Zubereitung nach Anspruch 12, dadurch gekennzeichnet, daß sie Zink-Ionen enthält, vorzugsweise von etwa 2 $\mu$g bis etwa 2mg Zink pro ml, am bevorzugtesten von etwa 5 $\mu$g bis 200 $\mu$g Zink pro ml.

**14.** Zinklösung zur Verwendung bei der Herstellung einer Lösung mit verlängerter Wirkung gemäß Anspruch 12, indem sie mit einer Lösung vermischt wird, die eine Verbindung von Formel I, angegeben in Anspruch 11, enthält, wobei diese Zinklösung vorzugsweise zwischen etwa 10 $\mu$g und 20 mg Zink pro ml enthält.

**15.** Verfahren zur Herstellung einer Lösung gemäß Anspruch 12, dadurch gekennzeichnet, daß eine Lösung einer Verbindung von Formel I, angegeben in Anspruch 12, die fakultativ Zink enthält, mit einer Zinklösung vermischt wird, die fakultativ eine Verbindung von Formel I, angegeben in Anspruch 12, enthält, was eine Mischung mit einer Zinkkonzentration von bis zu 2 mg/ml ergibt, und vorzugsweise der Gehalt an Zink in jeder der zwei Lösungen geringer ist als etwa 4 mg/ml.

**16.** Verfahren zur Herstellung von Verbindungen der allgemeinen Formel I, angegeben in Anspruch 12, gekennzeichnet durch Transpeptidierung eines Insulinvorläufers der allgemeinen Formel II:

$$\begin{array}{l} X\text{-}B(26\text{-}22)\text{-}E^4\text{-}B(20\text{-}14)\text{-}E^3\text{-}B(12\text{-}1) \\ | \\ B(28\text{-}29)\text{-}(Q_q\text{-}T)_r\text{-}A(1\text{-}3)\text{-}E^1\text{-}A(5\text{-}16)\text{-}E^2\text{-}A(18\text{-}20)\text{-}W \end{array} \qquad (II)$$

wobei A und B Fragmente der A- und B-Ketten bezeichnen, angegeben durch Zahlen in Klammern, Q eine Peptidkette mit q Aminosäuren ist, q eine ganze Zahl von 0 bis 33 ist, T Lys oder Arg ist und r Null oder Eins ist und $E^1$, $E^2$, $E^3$, $E^4$, W und X jeweils sind, wie in Anspruch 12 definiert, mit einer Verbindung der allgemeinen Formel III:

$H\text{-}Y_m\text{-}Z_n\text{-}R$,

in der Y, Z, R, m und n jeweils sind, wie oben definiert, und wobei Seitenkettenaminogruppen und -hydroxygruppen in Y und Z fakultativ mit Amino- und Hydroxy-Schutzgruppen blockiert sind, unter Verwendung von Trypsin oder einem trypsinähnlichen Enzym als einem Katalysator.

**Revendications**

**1.** Composés de la formule générale I

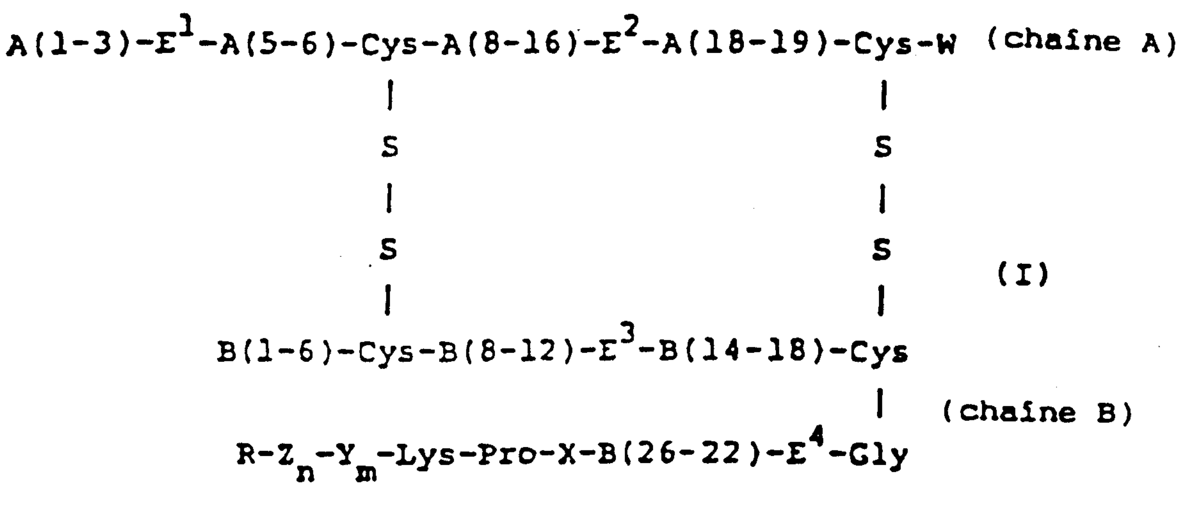

(I)

dans laquelle les lettres A et B suivies des chiffres entre parenthèses désignent les fragments peptides des chaînes A et B respectivement, désignés par les chiffres entre parenthèses, $E^1$, $E^2$, $E^3$ et $E^4$ sont identiques ou différents représentant chacun l'acide glutamique ou un résidu d'acide aminé neutre qui peut être codé par des séquences nucléotides, X représente un résidu L-lysine, L-thréonine ou L-arginine, Y et Z sont identiques ou différents et chacun représente un résidu d'acide aminé dans lequel tout groupe amino à chaîne latérale peut être acylé et dans lequel tout groupe hydroxy à chaîne latérale peut être alcoylé, et m et n sont identiques ou différents et représentent chacun 0 ou 1, et R représente hydroxy ou un résidu amino ou ester qui bloque le groupe carboxyle en position C terminale de la chaîne B et W représente un résidu d'acide aminé sauf l'asparagine, à condition qu'au moins l'un des six résidus d'acide aminé $E^1$, $E^2$, $E^3$ et $E^4$, W et X soit différent des résidus d'acide

aminé présents dans les positions correspondantes de l'insuline humaine et en ce qu'au moins l'un des huit résidus d'acide aminé $E^1$, $E^2$, $E^3$ et $E^4$, W, X, Y et Z et le groupe R sont choisis de sorte que le composé de formule I possède au moins une charge supérieure à l'insuline humaine à une valeur de pH de 7.

**2.** Composé selon la revendication 1, caractérisé en ce que X est un résidu arginine ou lysine.

**3.** Composés selon l'une quelconque des revendications précédentes, caractérisés en ce que $E^2$ et $E^3$ sont chacun un résidu glutamine.

**4.** Composés selon l'une quelconque des revendications précédentes, caractérisés en ce que Y et/ou Z est un résidu d'acide aminé de base dans lequel le groupe aminé de chaîne latérale est facultativement acylé (m = 1).

**5.** Composés selon l'une quelconque des revendications précédentes, caractérisés en ce que n est 0 et Y est un résidu d'acide aminé de base (m = 1).

**6.** Composés selon l'une quelconque des revendications précédentes, caractérisés en ce que Y et Z sont tous deux des résidus d'acide aminé de base (m = 1, n = 1).

**7.** Composés selon l'une quelconque des revendications précédentes, caractérisés en ce que R est un groupe de formule générale $-NR^1R^2$ dans laquelle $R^1$ et $R^2$ sont identiques ou différents et représentent chacun l'hydrogène ou un alkyle inférieur, et de préférence R est $-NH_2$.

**8.** Composés selon l'une quelconque des revendications 1 à 6, caractérisés en ce que R est un alcoxy inférieur, de préférence un butyloxy tertiaire.

**9.** Composés selon l'une quelconque des revendications 1 à 6, caractérisés en ce que R est un résidu d'un lactame qui contient de préférence moins de 8 atomes dans le cycle lactame.

**10.** Composés selon l'une quelconque des revendications précédentes, caractérisés en ce que W est Gly, Ser, Thr, Ala, His, Asp ou hSer.

**11.** Composé selon la revendication 1, caractérisé en ce qu'il s'agit
$Asp^{A21}$, $Arg^{B27}$, $Lys^{B30}$-$NH_2$ insuline humaine,
$Asp^{A21}$, $Arg^{B27}$ insuline humaine,
$Gly^{A21}$, $Arg^{B27}$ insuline humaine,
$Gln^{A17}$, $Ser^{A21}$, $Arg^{B27}$ insuline humaine,
$Gln^{A17}$, $Thr^{A21}$, $Arg^{B27}$ insuline humaine,
$Gln^{A17}$, $His^{A21}$, $Arg^{B27}$ insuline humaine,
$Gln^{A17}$, $Asp^{A21}$, $Arg^{B27}$ insuline humaine,
$Gln^{A17}$, $Gly^{A21}$, $Arg^{B27}$ insuline humaine,
$Gln^{A17}$, $Ser^{A21}$, $Gln^{B13}$ insuline humaine,
$Gln^{A17}$, $Thr^{A21}$, $Gln^{B13}$ insuline humaine,
$Gln^{A17}$, $Ala^{A21}$, $Gln^{B13}$ insuline humaine,
$Gln^{A17}$, $His^{A21}$, $Gln^{B13}$ insuline humaine,
$Gln^{A17}$, $Asp^{A21}$, $Gln^{B13}$ insuline humaine,
$Gln^{A17}$, $Gly^{A21}$, $Gln^{B13}$ insuline humaine,
$Arg^{B27}$, $Ser^{A21}$, $Gln^{B13}$ insuline humaine,
$Arg^{B27}$, $Thr^{A21}$, $Gln^{B13}$ insuline humaine,
$Arg^{B27}$, $Ala^{A21}$, $Gln^{B13}$ insuline humaine,
$Arg^{B27}$, $His^{A21}$, $Gln^{B13}$ insuline humaine,
$Arg^{B27}$, $Asp^{A21}$, $Gln^{B13}$ insuline humaine,
$Arg^{B27}$, $Gly^{A21}$, $Gln^{B13}$ insuline humaine,
$Gln^{A17}$, $Ser^{A21}$, $Lys^{B27}$ insuline humaine,
$Gln^{A17}$, $Thr^{A21}$, $Lys^{B27}$ insuline humaine,
$Gln^{A17}$, $Ala^{A21}$, $Lys^{B27}$ insuline humaine,
$Gln^{A17}$, $His^{A21}$, $Lys^{B27}$ insuline humaine,

$Gln^{A17}$, $Asp^{A21}$, $Lys^{B27}$ insuline humaine,
$Gln^{A17}$, $Gly^{A21}$, $Lys^{B27}$ insuline humaine,
$Gln^{B13}$, $Ser^{A21}$, $Lys^{B27}$ insuline humaine,
$Gln^{B13}$, $Thr^{A21}$, $Lys^{B27}$ insuline humaine,
$Gln^{B13}$, $Ala^{A21}$, $Lys^{B27}$ insuline humaine,
$Gln^{B13}$, $His^{A21}$, $Lys^{B27}$ insuline humaine,
$Gly^{A21}$, $Lys^{B27}$, $Thr^{B30}$-$NH_2$ insuline humaine,
$Ser^{A21}$, $Lys^{B27}$, $Thr^{B30}$-$NH_2$ insuline humaine,
$Thr^{A21}$, $Lys^{B27}$, $Thr^{B30}$-$NH_2$ insuline humaine,
$Ala^{A21}$, $Lys^{B27}$, $Thr^{B30}$-$NH_2$ insuline humaine,
$His^{A21}$, $Lys^{B27}$, $Thr^{B30}$-$NH_2$ insuline humaine,
$Asp^{A21}$, $Lys^{B27}$, $Thr^{B30}$-$NH_2$ insuline humaine,
$Gly^{A21}$, $Arg^{B27}$, $Thr^{B30}$-$NH_2$ insuline humaine,
$Ser^{A21}$, $Arg^{B27}$, $Thr^{B30}$-$NH_2$ insuline humaine,
$Thr^{A21}$, $Arg^{B27}$, $Thr^{B30}$-$NH_2$ insuline humaine,
$Ala^{A21}$, $Arg^{B27}$, $Thr^{B30}$-$NH_2$ insuline humaine,
$His^{A21}$, $Arg^{B27}$, $Thr^{B30}$-$NH_2$ insuline humaine,
$Asp^{A21}$, $Arg^{B27}$, $Thr^{B30}$-$NH_2$ insuline humaine,
$Gln^{B13}$, $Gly^{A21}$, $Arg^{B27}$, $Thr^{B30}$-$NH_2$ insuline humaine,
$Gln^{B13}$, $Ser^{A21}$, $Arg^{B27}$, $Thr^{B30}$-$NH_2$ insuline humaine,
$Gln^{B13}$, $Thr^{A21}$, $Arg^{B27}$, $Thr^{B30}$-$NH_2$ insuline humaine,
$Gln^{B13}$, $Ala^{A21}$, $Arg^{B27}$, $Thr^{B30}$-$NH_2$ insuline humaine,
$Gln^{B13}$, $His^{A21}$, $Arg^{B27}$, $Thr^{B30}$-$NH_2$ insuline humaine,
$Gln^{B13}$, $Asp^{A21}$, $Arg^{B27}$, $Thr^{B30}$-$NH_2$ insuline humaine,
$Gln^{B13}$, $Gly^{A21}$, $Lys^{B27}$, $Thr^{B30}$-$NH_2$ insuline humaine,
$Gln^{B13}$, $Ser^{A21}$, $Lys^{B27}$, $Thr^{B30}$-$NH_2$ insuline humaine,
$Gln^{B13}$, $Thr^{A21}$, $Lys^{B27}$, $Thr^{B30}$-$NH_2$ insuline humaine,
$Gln^{B13}$, $Ala^{A21}$, $Lys^{B27}$, $Thr^{B30}$-$NH_2$ insuline humaine,
$Gln^{B13}$, $His^{A21}$, $Lys^{B27}$, $Thr^{B30}$-$NH_2$ insuline humaine,
$Gln^{B13}$, $Asp^{A21}$, $Lys^{B27}$, $Thr^{B30}$-$NH_2$ insuline humaine,
$Ser^{A21}$, $Lys^{B27}$ insuline humaine,
$Thr^{A21}$, $Lys^{B27}$ insuline humaine,
$Ala^{A21}$, $Lys^{B27}$ insuline humaine,
$His^{A21}$, $Lys^{B27}$ insuline humaine,
$Asp^{A21}$, $Lys^{B27}$ insuline humaine,
$Gly^{A21}$, $Lys^{B27}$ insuline humaine,
$Ser^{A21}$, $Arg^{B27}$ insuline humaine,
$Thr^{A21}$, $Arg^{B27}$ insuline humaine,
$Ala^{A21}$, $Arg^{B27}$ insuline humaine,
$His^{A21}$, $Arg^{B27}$ insuline humaine,
$Gln^{B13}$, $Asp^{A21}$, $Lys^{B27}$ insuline humaine,
$Gln^{B13}$, $Gly^{A21}$, $Lys^{B27}$ insuline humaine ou
$hSer^{A21}$, $Arg^{B27}$, $Thr^{B30}$-$NH_2$ insuline humaine.

**12.** Solutions injectables à action d'insuline prolongée, caractérisées en ce qu'elles contiennent un composé de formule générale I

```
A(1-3)-E1-A(5-6)-Cys-A(8-16)-E2-A(18-19)-Cys-W   (chaîne A)
                   |                        |
                   S                        S
                   |                        |
                   S                        S       (I)
                   |                        |
        B(1-6)-Cys-B(8-12)-E3-B(14-18)-Cys
                                            |     (chaîne B)
        R-Zn-Ym-Lys-Pro-X-B(26-22)-E4-Gly
```

dans laquelle les lettres A et B suivies des chiffres entre parenthèses désignent les fragments peptides des chaînes A et B respectivement, désignés par les chiffres entre parenthèses, $E^1$, $E^2$, $E^3$ et $E^4$ sont identiques ou différents représentant chacun l'acide glutamique ou un résidu d'acide aminé neutre qui peut être codé par des séquences nucléotides, X représente un résidu L-lysine, L-thréonine ou L-arginine, Y et Z sont identiques ou différents et chacun représente un résidu d'acide aminé dans lequel tout groupe amino à chaîne latérale peut être acylé et dans lequel tout groupe hydroxy à chaîne latérale peut être alcoylé, et m et n sont identiques ou différents et représentent chacun 0 ou 1, et R représente hydroxy ou un résidu amino ou ester qui bloque le groupe carboxyle en position C terminale de la chaîne B et W représente un résidu d'acide aminé sauf l'asparagine, à condition qu'au moins l'un des six résidus d'acide aminé $E^1$, $E^2$, $E^3$ et $E^4$, W et X soit différent des résidus d'acide aminé présents dans les positions correspondantes de l'insuline humaine et en ce qu'au moins l'un des huit résidus d'acide aminé $E^1$, $E^2$, $E^3$ et $E^4$, W, X, Y et Z et le groupe R sont choisis de sorte que le composé de formule I possède au moins une charge supérieure à l'insuline humaine à une valeur de pH de 7.

**13.** Préparation selon la revendication 12, caractérisée en ce qu'elle contient des ions zinc, de préférence d'environ 2 µg jusqu'à 2 mg de zinc par ml, de façon plus préférée d'environ 5 µg jusqu'à 200 µg de zinc par ml.

**14.** Solution de zinc pour l'utilisation dans la préparation d'une solution à action plus prolongée selon la revendication 12, en la mélangeant avec une solution contenant un composé de formule I désigné dans la revendication 11, laquelle solution de zinc contient de préférence entre environ 10 µg et 20 mg de zinc par ml.

**15.** Procédé pour la préparation d'une solution selon la revendication 12, caractérisé en ce qu'une solution d'un composé de formule I désigné dans la revendication 12, contenant facultativement du zinc est mélangé avec une solution de zinc contenant facultativement un composé de formule I désigné dans la revendication 12, donnant un mélange avec une concentration de zinc jusqu'à 2 mg/ml et de préférence la teneur en zinc dans chacune des deux solutions est inférieure à environ 4 mg/ml.

**16.** Procédé pour la préparation de composés de la formule générale I désigné dans la revendication 12, caractérisé en ce que l'on transpeptidate un précurseur d'insuline de la formule générale II :

```
X-B(26-22)-E4-B(20-14)-E3-B(12-1)
|                                                        (II)
B(28-29)-(Qq-T)r-A(1-3)-E1-A(5-16)-E2-A(18-20)-W
```

dans laquelle A et B désignent les fragments des chaînes A et B désignés par les numéros entre

parenthèses, Q est une chaîne peptide avec des acides aminés q, q est un nombre entier de 0 à 33, T est Lys ou Arg, et r est égal à 0 ou à 1 et $E^1$, $E^2$, $E^3$, $E^4$, W et X sont chacun tels que définis dans la revendication 12, avec un composé de la formule générale III :

$H\text{-}Y_m\text{-}Z_n\text{-}R$ (III)

dans laquelle Y, Z, R, m et n sont chacun tels que définis ci-dessus, et dans laquelle les groupes aminés à chaîne latérale et les groupes hydroxy en Y et Z sont facultativement bloqués par des groupes protecteurs amino et hydroxy en utilisant une enzyme trypsine ou analogue à la trypsine comme catalyseur.